(19) Europäisches Patentamt
European Patent Office
Office européen des brevets

(11) Publication number: 0 020 172
A2

# (12) EUROPEAN PATENT APPLICATION

(21) Application number: 80301832.4

(22) Date of filing: 02.06.80

(51) Int. Cl.³: A 01 N 57/16

(30) Priority: 05.06.79 US 45811
11.01.80 US 111506

(43) Date of publication of application: 10.12.80
Bulletin 80/25

(84) Designated Contracting States: DE FR GB IT

(71) Applicant: FMC Corporation, 2000 Market Street, Philadelphia Pennsylvania 19103 (US)

(72) Inventor: James, Donald Richard, 169 Goldenrad DR, Hercules CA 94547 (US)

(74) Representative: Crampton, Keith John Allen et al, D YOUNG & CO 10 Staple Inn, London WC1V 7RD (GB)

## (54) Nematicidal haloethenylthiophenes and their use.

(57) A method is disclosed for the control of nematodes in agricultural crops which comprises applying to the situs of infestation a compound of the formula

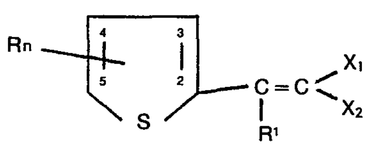

wherein $X_1$ and $X_2$ are each halogen atoms; $R^1$ is hydrogen, lower alkyl, or di(lower)alkyl phosphate; each R is hydrogen, halogen, lower alkyl or nitro; and n is 0, 1, or 2. Preparation and nematicidal utility for the compounds are described and exemplified. When $R^1$ is other than hydrogen or at least one R on the thiophene ring is nitro, advantageously at the 3 or 5 position of the thiophene ring, the compounds of the above formula are novel.

EP 0 020 172 A2

NEMATICIDAL HALOETHENYLTHIOPHENES

The present invention relates to a method and composition for controlling nematodes and to novel nematicidal compounds. More particularly, the invention relates to dihaloethenylthiophenes useful as nematicides for agricultural crops.

Dihaloethenylthiophenes are known to be useful as intermediates for the preparation of pharmaceuticals. German Offenlegungsschrift 2810262, published September 28, 1978 generically describes compounds useful for that purpose and specifically exemplifies 2-(2,2-dichloroethenyl)thiophene. Certain ethenylthiophenes have been reported by Handele to have nematicidal activity, for example, 1,2-di(2-thienyl)-ethenes and 1-phenyl-2-thienyl ethenes. See M.J. Handele, SYNTHESE, SPECTRA EN NEMATICIDE ACTIVITEIT VAN 1,2-DITHIENYLETHENEN EN 1-FENYL-2-THIENYLETHENEN, Thesis, University of Utrecht, 1971. Certain dihaloethenylnitrofurans are also said to have nematicidal activity. See, for example, U.S. Patent 4,024,161, issued May 17, 1977. It has now been found that the dihaloethenylthiophenes described below are excellent nematicides for use in agricultural crops.

The present invention thus provides a method and composition for controlling nematodes in agricultural crops by applying to the locus of infestation a nematicidally effective amount of a compound of the formula

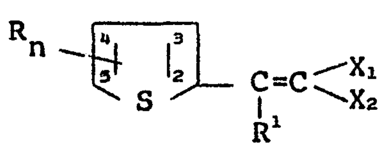

wherein each X is a halogen atom; each R is halogen, hydrogen, lower alkyl, or nitro;
n is 0, 1 or 2; and
$R^1$ is hydrogen, lower alkyl, or di(lower)alkyl phosphate.

Particularly effective as nematicides are compounds in which at least one R is other than hydrogen, especially at the 3 or 5 position of the thiophene ring, preferably

nitro or lower alkyl at position 5 of the ring. Also of particular interest are compounds in which $R^1$ is other than hydrogen.

The novel compounds of this invention are those described above in which $R^1$ is other than hydrogen or in which at least one R on the thiophene ring is nitro, advantageously at the 3 or 5 position of the thiophene ring, preferably nitro at the position 5 of the ring.

Throughout the specification and claims the term "lower", as applied to a hydrocarbyl radical means having 1-4 carbon atoms, and the term "halogen" or "halo" means bromine, chlorine or fluorine, preferably bromine or chlorine.

The nematicides of this invention, like most agricultural chemicals, are generally not applied full strength, but are formulated with agriculturally acceptable carriers normally employed for facilitating the dispersion of active ingredients, various additives, and optionally with other active ingredients, recognizing the accepted fact that the formulation and mode of application of a toxicant may affect the activity of the material. The present compounds may be applied, for example, to the soil in which nematode control is desired as granules or powders or liquids, the choice of application varying, of course, with the nematode species and environmental factors present at the particular locus of infestation. Thus, the compounds may be formulated as granules of various sizes, as dusts, as wettable powders, as emulsifiable concentrates, as solutions, as dispersions, as controlled release compositions, and the like. Some of the compounds may also be applied to the exposed leaves and stems for control of soil infestations of nematodes.

A typical formulation may vary widely in concentration of the active ingredient depending on the particular agent used, the additives and carriers used, other active ingredients, and the desired mode of application. With

due consideration of these factors, the active ingredient of a typical formulation may, for example, be suitably present at a concentration of from about 0.5% up to about 99.5% by weight of the formulation. Substantially inactive ingredients such as adjuvants and carriers may comprise from about 99.5% by weight to as low as about 0.5% by weight of the formulation. Surface active agents, if employed in the formulation, may be present at various concentrations, suitably in the range of 1 to 30% by weight. Provided below is a general description of exemplary types of formulations which may be employed for dispersion of the nematicides of the present invention.

Dusts are admixtures of the active ingredient with finely divided solid carriers and/or diluents such as talc, natural clays, kieselguhr, pyrophyllite, chalk, diatomaceous earths, calcium phosphates, calcium and magnesium carbonates, sulfur, lime, flours, and other organic and inorganic solid carriers. These finely divided formulations generally have an average particle size of less than about 50 microns (325 mesh, U.S. Sieve Series ASTM-E-11-61). In most cases, the active ingredient will be present in dust formulations at a concentration in the range of 1 to 15%, and occasionally from 1% to about 30%, the balance of the composition typically being agriculturally acceptable carrier or diluent.

Wettable powders, also useful formulations for these nematicides, are in the form of finely divided particles which disperse readily in water or other liquid vehicles. The wettable powder is ultimately applied to the soil or plant as a dry dust or a dispersion in water or other liquid. Typical carriers for wettable powders include fuller's earth, kaolin clays, silicas, and other highly absorbent or adsorbent inorganic diluents. The concentration of active ingredient in wettable powders is dependent upon physical properties of the active ingredient and the absorbency characteristics of the carrier. Liquids and low melting solids (mp $< 100°C$)

are suitably formulated in the concentration range of 5 to 50% by weight, usually from 10 to 30%; high melting solids (mp > 100°C) being formulated in the range of 5 to 95% by weight, usually 50 to 85%. An agriculturally acceptable carrier or diluent, frequently including a small amount of a surfactant to facilitate wetting dispersion and suspension, accounts for the balance of the formulation.

Granules are admixtures of the active ingredients with solids of particle sizes generally in the range of 4 to 100 mesh (U.S. Sieve Series). Granular formulations may employ hard core materials such as sands and other silicates, mineral carbonates, sulfates or phosphates and the like, or porous cores such as attapulgite clays, fuller's earth, kieselguhr, chalk, diatomaceous earths, ground corn cobs, wood dusts and the like. Impregnating or binding agents such as aliphatic and aromatic petroleum solvents, alcohols, ethers, ketones, esters, vegetable oils, polyvinyl acetates, polyvinyl alcohols, dextrins, sugars and the like are commonly used to aid in coating or impregnating the solid carriers with the active ingredient. Emulsifying agents, wetting agents, dispersing agents, and other additives known in the art may also be added.

A typical granular formulation may suitably contain from about 1% to about 50% by weight active ingredient and 99% to 50% by weight of inert materials.

Microencapsulated or other controlled release formulations may also be used with nematicides of this invention for control of nematodes.

Emulsifiable concentrates (EC's) are homogeneous liquid compositions, usually containing the active ingredient dissolved in a liquid carrier. Commonly used liquid carriers include xylene, heavy aromatic naphthas, isophorone, and other nonvolatile or slightly volatile organic solvents. For application of the nematicide, these concentrates are dispersed in water,

or other liquid vehicle, forming an emulsion, and are normally applied as a spray to the area to be treated. The concentration of the essential active ingredient in EC's may vary according to the manner in which the composition is to be applied, but, in general, is in the range of 0.5 to 95%, frequently 10 to 80%, by weight of active ingredient, with the remaining 99.5% to 5% being surfactant and liquid carrier.

Flowables are similar to EC's except that the active ingredient is suspended in a liquid carrier, generally water. Flowables, like EC's, may include a small amount of a surfactant, and contain active ingredient in the range of 0.5 to 95%, frequently from 10 to 50%, by weight of the composition. For application, flowables may be diluted in water or other liquid vehicle, and are normally applied as a spray to the area to be treated.

Typical wetting, dispersing or emulsifying agents used in nematicidal formulations include, but are not limited to, the alkyl and alkylaryl sulfonates and sulfates and their sodium salts; alkylaryl polyether alcohols, sulfated higher alcohols; polyethylene oxides; sulfonated animal and vegetable oils; sulfonated petroleum oils; fatty acid esters of polyhydric alcohols and the ethylene oxide addition products of such esters; and the addition product of long-chain mercaptans and ethylene oxide. Many other types of useful surface-active agents are available in commerce. The surface-active agent, when used, normally comprises from 1 to 15% by weight of the nematicidal composition.

Other useful formulations include simple solutions of the active ingredient in a relatively non-volatile solvent such as corn oil, kerosene, propylene glycol, or other organic solvents. This type of formulation is particularly useful for ultra low volume application.

The concentration of the nematicide in use dilution is normally in the range of about 2% to about 0.1%.

Many variations of spraying, dusting, soil-incorporated, and controlled or slow release compositions in the art may be used by substituting or adding a compound of this invention into compositions known or apparent to the art.

Nematicidal compositions may be formulated and applied with other suitable active ingredients, including other nematicides, insecticides, acaricides, fungicides, plant regulators, herbicides, fertilizers, and so forth.

In applying the foregoing chemicals, an effective nematicidal amount must be applied. While the application rate will vary widely depending on the choice of compound, the formulation and mode of application, the plant species being protected and the planting density, a suitable use rate may be in the range of 0.5 to 25 kg/hectare, preferably 1 to about 20 kg/hectare. Trees and vines for example may require at least 5 kg/hectare whereas annuals such as corn may require considerably lower rates of application, for example 1 to 5 kg/hectare.

The following illustrate the general methods for preparing the compounds of this invention.

A. 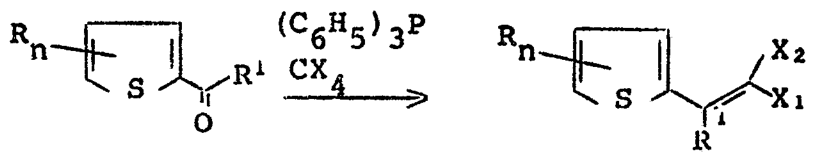


B. 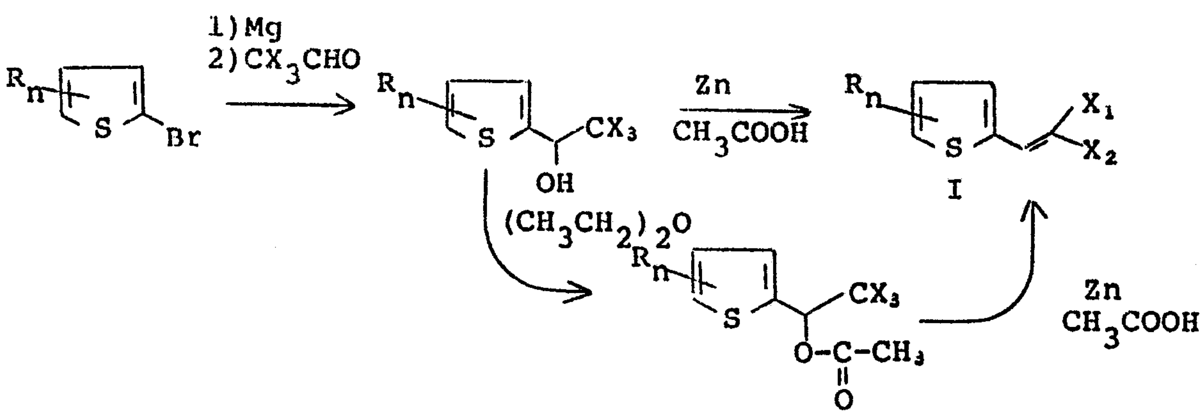

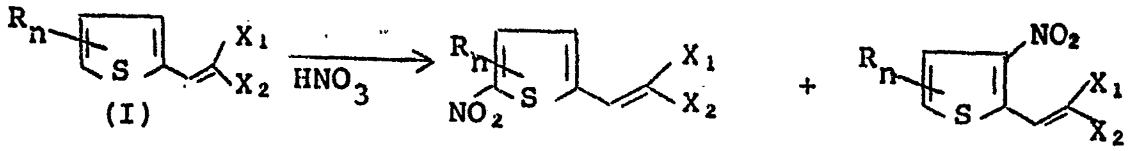


(I)

C.

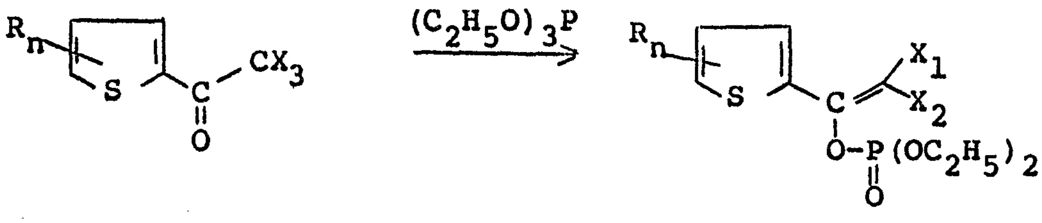


Examples 1 through 4 illustrate preparation of the compounds of this invention by Route A.

Example 1

Synthesis of 2-(2,2-dibromoethenyl)-thiophene

Triphenylphosphine (42 g, 0.16 mole), zinc dust (10.5 g, 0.16 mole), and carbon tetrabromide (53.1 g, 0.16 mole) were placed in a 3-necked flask equipped with a magnetic stirrer and dry nitrogen inlet. Anhydrous methylene chloride (200 ml) was added under a flow of nitrogen and the resulting reaction mixture stirred at room temperature for 24 hours. After the sludge which had formed was broken up, 2-thiophenecarboxaldehyde (7.2 g, 0.064 mole) was added in one portion under a nitrogen blanket, and stirring was continued for an additional 2 hours. The resulting mixture was diluted with an equal volume of hexane and filtered. The solids were then extracted with five 200 ml portions of hexane. The combined filtrates were then refiltered, and solvent was removed utilizing a rotary evaporator. The resulting black solid was passed through a silica gel column with hexane to give 12.7 g of 2-(2,2-dibromoethenyl)thiophene as a tan solid, m.p. 54-55°C.

Analysis calculated

for $C_6H_4Br_2S$: C 26.89; H 1.50; Br 59.64;

Found: C 26.74; H 1.69; Br 59.61.

Example 2

Synthesis of 2-(2,2-dibromoethenyl)-3-methylthiophene

In a manner similar to Example 1, the reaction of triphenylphosphine, zinc dust, and carbon tetrabromide in dichloromethane with 3-methyl-2-thiophenecarboxaldehyde produced 2-(2,2-dibromoethenyl)-3-methylthiophene b.p. 113°C/27 pa.

Elemental analysis calculated

for $C_7H_6Br_2S$: C 29.81; H 2.14; Br 56.67;

Found: C 29.77; H 2.16; Br 56.76.

Example 3

Synthesis of 2-(2,2-dibromoethenyl)-5-bromothiophene

In a manner similar to Example 1, the reaction of triphenylphosphine, zinc dust and carbon tetrabromide in methylene chloride with 5-bromo-2-thiophenecarboxaldehyde produced 5-bromo-2-(2,2-dibromoethenyl)-5-bromothiophene, m.p. 64-67°C. A sample was then sublimed at 60°C/27 pa, m.p. 66.5-68°C.

Elemental analysis calculated

for $C_6H_3Br_3S$: C 20.77; H 0.87; Br 69.11;

Found: C 20.90; H 1.13; Br 69.19.

Example 4

Synthesis of 2-(2,2-dichloro-1-methylethenyl)thiophene

A stirred mixture of 8.56 g (0.068 mole) of 2-acetylthiophene and 33.0 g (0.124 mole) of triphenylphosphine in 150 ml of carbon tetrachloride was heated at reflux for four hours. An additional 33.0 g (0.124 mole) of triphenylphosphine was then added, and heating was continued for two hours. The reaction mixture was cooled, filtered and the filtered solids were washed with diethyl ether. Diethyl ether and a small amount of carbon tetrachloride were removed from the filtrate by evaporation. Dissolved triphenylphosphine was precipitated by the addition of low boiling petroleum ether and the precipitate separated by filtration. The solvents were removed from the filtrate by evaporation to give an oil. Fractional distillation of the oil gave crude

2-(2,2-dichloro-1-methylethenyl)thiophene (b.p. 66°-68°C/27 pa). Redistillation gave 2.5 g 2-(2,2-dichloro-1-methylethenyl)thiophene, b.p. 121°-124°C/2.7 Kpa.

Elemental analysis

calculated for $C_7H_6Cl_2S$: C 43.54; H 3.13; Cl 36.72;

Found: C 43.61; H 3.41; Cl 36.64.

Examples 5 through 8 illustrate the preparation according to Route B.

Example 5

Synthesis of 2-(2,2-dichloroethenyl)thiophene

2,2,2-Trichloro-1-(2-thienyl)-1-ethanol (21.0 g, 0.091 mole), prepared as described by Floutz, J.A.C.S. 71 2859-60 (1949) and 0.5 ml trifluoroacetic acid in 42.0 g (0.411 mole) acetic anhydride was heated at reflux for three hours. The reaction mixture was cooled, poured into 1500 ml of stirred ice water. The resulting precipitate was separated by filtration and washed with water. Recrystallization gave 22.1 g of 2,2,2-trichloro-1-(2-thienyl)ethylacetate. The nmr spectrum and infrared spectrum were consistent with the assigned structure.

To a stirred solution of 2.0 g (0.007 mole) of 2,2,2-trichloro-1-(2-thienyl)ethylacetate in 10 ml of acetic acid was added 0.72 g (0.01 mole) of zinc dust. The reaction mixture was stirred at room temperature for 18 hours. An additional 0.2 g of zinc dust was then added and the reaction mixture stirred over night. The reaction mixture was filtered and the filter cake washed with ethanol. The combined filtrates were poured into 300 ml of water. The resultant precipitate was filtered and sublimed (100°C/10 mm Hg) to give 1.0 g of 2-(2,2-dichloroethenyl)thiophene. The nmr spectrum was consistent with the assigned structure.

Example 6

Synthesis of 2-(2,2-dichloroethenyl)-5-nitrothiophene and 2-(2,2-dichloroethenyl)-3-nitrothiophene

To a stirred cold solution of 4.0 g (0.022 mole) of

2-(2,2-dichloroethenyl)thiophene in 65 ml of acetic anhydride was added a solution of 4.4 g (0.07 mole) of nitric acid and 22 ml of acetic acid. After complete addition the reaction mixture was warmed to room temperature and stirred overnight. The reaction mixture was poured into 200 ml of ice water and extracted with three 150 ml portions of diethyl ether. The combined extracts were washed with two 100 ml portions of water, once with 100 ml of a saturated sodium chloride solution, dried with anhydrous magnesium sulfate, and filtered. The solvent was removed from the filtrate to give a solid. Recrystallization of the solid from methanol and water gave 1.3 g of solid (m.p. 89-90°C). The mother liquor was cooled in a freezer overnight. The solid was sublimed (110°C/39.9 pa) to yield a solid (m.p. 83°-86°C). Recrystallization from methanol gave 2-(2,2-dichloroethenyl)-5-nitrothiophene (m.p. 90°-91°C). The nmr spectrum and infrared spectrum were consistent with the proposed structure.

Elemental analysis calculated for $C_6H_3Cl_2NO_2S$:

C 32.16; H 1.35; N 6.25; Cl 31.65; S 14.31;

Found: C 32.29; H 1.54; N 6.53; Cl 31.86; S 14.16.

The cooled mother liquor contained a precipitate which was recovered by filtration. The solid was subjected to column chromatography on silica gel using 5% ethylacetate in heptane as eluent to give 0.23 g of 2-(2,2-dichloroethenyl)-5-nitrothiophene and 0.23 g of 2-(2,2-dichloroethenyl)-3-nitro-thiophene (m.p. 56°-60°C). The nmr spectrum was consistent with the proposed structure.

Elemental analysis calculated for $C_6H_3Cl_2NO_2S$:

C 32.16; H 1.35; N 6.25; Cl 31.65; S 14.31;

Found: C 31.85; H 1.35; N 6.18; Cl 32.63; S 14.68.

Example 7

Synthesis of 2-(2,2-dichloroethenyl)-5-bromothiophene

To a stirred ice cooled mixture of 54.0 g (0.826 mole) of zinc in 300 ml of acetic acid was added slowly

128.0 g (0.413 mole) of 2,2,2-trichloro-1-(5-bromo-2-thienyl)ethanol (prepared from 2,5-dibromothiophene by the method of Floutz, as in Example 5). The reaction mixture was warmed to room temperature and stirred for four days, diluted with 350 ml of water, and extracted with three 200 ml portions of ethyl acetate. The combined extracts were washed with two 100 ml portions of water, dried with anhydrous magnesium sulfate, and filtered. The solvent was removed from the filtrate to give a dark liquid. Distillation of the liquid at reduced pressure gave 33.96 g of a slightly colored liquid (b.p. 93°-105°C/66.5 pa). The liquid was subjected to column chromatography on silica gel using pentane as the eluant to yield 30.6 g of a slightly colored liquid. A three gram sample of this liquid was subjected to column chromatography to yield 2.5 g of crude liquid product. A sample of this liquid was distilled to yield a liquid which solidified (m.p. 39°-40°C). An elemental analysis of this solid was not consistent with the proposed structure. Another sample of the 2.5 g of crude liquid product was crystallized in methanol. The solid was recrystallized twice from methanol, and sublimed (65°-70°C/39.9 pa) to give 2-(2,2-dichloroethenyl)-5-bromothiophene (m.p. 39-40°C). The nmr spectrum and infrared spectrum were consistent with the proposed structure.

Elemental analysis calculated
for $C_6H_3BrCl_2S$: C 27.93; H 1.17; Br 30.98; Cl 27.49;
Found: C 27.92; H 1.36; Br 30.77; Cl 27.33.

## Example 8

### Synthesis of 2-(2,2-dichloroethenyl)-5-methylthiophene

To a stirred mixture of 100 ml of anhydrous carbon tetrachloride and 45.4 g (0.255 mole) of N-bromosuccinimide was added 25.0 g (0.255 mole) of 2-methylthiophene. The reaction mixture was heated at reflux for 2 1/2 hours, cooled to room temperature, then stirred overnight. The reaction mixture was filtered and the filter

cake washed with chloroform. The solvents were removed from the combined filtrates under reduced pressure to give a dark oil. The oil was distilled to give three fractions, 22.2 g, of a mixture of 2-bromo-5-methylthiophene and 2-bromomethylthiophene, and one fraction, 5.8 g, of 2-bromomethylthiophene. The distillates were combined and treated with 20.0 g (0.076 mole) of triphenylphosphine in 100 ml of anhydrous toluene at room temperature for 12 hours, diluted with n-pentane, then filtered. The filter cake was washed with n-pentane and the filtrate evaporated to give a liquid. The liquid was distilled under reduced pressure to give 10.8 g of 2-bromo-5-methylthiophene, b.p. 52-70°C/2 Kpa. The nmr spectrum and infrared spectra were consistent with the assigned structure.

The 2-bromo-5-methylthiophene was converted to the intermediate 2,2,2-trichloro-1-(5-methylthienyl)-ethanol, b.p. 105°C/0.01 mm Hg, in accordance with the method of Floutz (see Example 5). This intermediate (10.3 g, 0.042 mole) was then reacted with 3.0 g zinc in 20 ml of acetic acid to give, after workup, 2.95 g 2-(2,2-dichloroethenyl)-5-methylthiophene, b.p. 115°C-125°C/1.7 Kpa. The nmr spectrum and infrared spectrum were consistent with the proposed structure.

Elemental analysis calculated
for $C_7H_6Cl_2S$: C 43.54; H 3.13; Cl 36.72;
Found: C 43.44; H 3.17; Cl 33.92.

Example 9 illustrates preparation in accordance with Route C.

## Example 9

**Synthesis of 2,2-dichloro-1-(2-thienyl)ethenyl diethylphosphate**

Under a dry argon atmosphere a stirred solution of 18.0 g (0.1 mole) of trichloroacetyl chloride and 8.4 g (0.1 mole) of thiophene in 50 ml of methylene chloride was cooled to -30°C. Aluminum chloride, 13.0 g (0.1 mole), was added during a five minute period, causing

the temperature to rise to 0°C. The reaction mixture was warmed to room temperature. A liquid was decanted from the reaction mixture, leaving an oily solid. The solid was washed with diethyl ether and the diethyl ether decanted. The decanted liquids were combined and poured into 300 ml of ice water. The resulting two phase mixture was filtered, the organic phase of the filtrate was separated and the aqueous phase was extracted with 50 ml of ether. The combined organic phases were washed with water, dried with anhydrous magnesium sulfate, and filtered. The solvent was removed from the filtrate by evaporation to give 16.2 g of liquid. Fractional distillation of the liquid gave 7.8 g of 2,2,2-trichloro-1-(2-thienyl) ethanone, b.p. 63°-70°C/20 pa. The infrared spectrum was consistent with the assigned structure.

During a ten minute period 5.65 g (0.033 mole) of triethyl phosphite was added dropwise to 7.8 g (0.033 mole) of 2,2,2-trichloro-1-(2-thienyl)ethanone and the mixture was allowed to stand overnight. The reaction mixture was distilled to yield 9.28 g of 2,2-dichloro-1-(2-thienyl)ethenyl diethylphosphate, b.p. 132°C/9 pa. The nmr spectrum and infrared spectra were consistent with the proposed structure.

<u>Preparation of formulations for testing</u>

The compound of Example 1 was formulated and tested for nematicidal activity as a formulated material. The formulation used was a standard 5 wt. % dust formulation made up as follows:

| | |
|---|---|
| Active ingredient (100% active basis) | 5 parts |
| Base | 95 parts |
| 96%-attaclay | |
| 2%-highly purified sodium lignosulfonate (100%) | |
| 2%-powdered sodium alkylnaphthalenesulfonate (75%) | |

The mixture was ground to a fine powder.

Biological Tests

The formulation described above was tested for activity against root-knot nematode (*Meloidogyne incognita*) as follows:

Nematode Culture - Tomato seedlings with two large true leaves were transplanted into six inch clay pots containing steam-sterilized sandy soil. One week after transplanting, galled roots of nematode-infested tomato plants, with fully developed egg masses, were placed in three holes in the soil around the seedling roots. Holes were then closed with soil. The plants were allowed to grow until fully developed egg masses were formed (6 to 7 weeks after inoculation).

Inoculum Preparation - Infected tomato roots, containing egg masses, were cleaned under running tap water, cut into short pieces and comminuted with water in an electrical blender for 30 seconds. The shredded roots were poured onto layers of washed sand in a wooden flat. The flat was covered with plastic sheeting and kept at greenhouse temperatures for 3 to 7 days to allow about 50% of the larvae to hatch.

Preparation of Root-Knot Nematode Infested Soil - Samples of the infested soil prepared as described above were processed for nematodes by using the Caveness and Jensen centrifugal-sugar flotation extraction technique [Caveness, F.E. and Jensen, H.J., "Modification of the Centrifugal Flotation Technique for the Isolation and Concentration of Nematodes and their Eggs from Soil and Plant Tissue," Proc. Helm. Soc., Washington, 22, 87-89 (1955).]

A 500 mesh sieve was used to collect the nematodes and eggs, and their number was estimated under a stereomicroscope. Enough sand containing eggs and larvae was mixed with additional steam-sterilized sandy soil so that there were 600 to 800 root-knot nematode larvae and eggs per pot of soil (three inch diameter each, containing approximately 300 g soil). Depending on the total

amount of nematode infested soil needed, mixing was accomplished by use of a cement mixer for 5 minutes or the V-shaped rotary mixer for 60 seconds.

Soil so infected was used for soil incorporated and systemic nematicidal studies within two days of preparation. For the soil incorporation tests the formulated compounds to be tested for nematicidal activity were incorporated in the root-knot nematode infested potting soil to give soil treatment at a rate of 25 ppm. (weight chemical/volume soil). Young tomato plants were planted in this soil in three inch pots. At the end of two weeks the roots of all plants were examined and rated in comparison to untreated checks. For the systemic test an aqueous acetonic solution (2400 ppm) was sprayed on the leaves and stems of plants planted in inoculated soil. For this test readings were taken at 1 and 7 days.

The rating system used to report results is as follows:

Knot Index

4 - No control - amount of swellings equivalent to that developed on the roots of the untreated check plants.

3 - Amount of sellings 25% less than that developed on the roots of the untreated check plants.

2 - Amount of swellings 50% less than that developed on the roots of the untreated check plants.

1 - Amount of swellings 75% less than that developed on the roots of the untreated check plants.

0 - No swellings - complete control.

Control Treatments - The untreated check plants were treated in the same manner as those treated with the active ingredient. The formulation base, without active ingredient, was added to the soil for untreated plants and separate untreated plants were used to detect the effects, if any, of chemicals in the formulation base. Each test series also included a formulation of carbofuran, a known nematicide, as a standard for

comparison.

Results - The results of the tests are shown in Table I. High levels of activity in the soil incorporated test were exhibited by most of the exemplified compounds. One of the compounds showed excellent systemic activity and another showed some systemic control after seven days.

Table I

| Compound of Example | Soil Incorporation Rate (ppm) | Soil Incorporation Knot Index[1] | Systemic Rate (ppm) | Systemic Knot Index Day 1 | Systemic Knot Index Day 7 |
|---|---|---|---|---|---|
| 1 | 25 | 0.0 | 2400 | 4.0 | 4.0 |
| 2 | 25 | 0.3 | 2400 | 4.0 | 4.0 |
| 3 | 25 | 0.1 | 2400 | 4.0 | 4.0 |
| 4 | 25 | 1.8 | 2400 | 4.0 | 3.0 |
| 5 | 25 | 1.0 | 2400 | 4.0 | 4.0 |
| 6(a)[2] | 25 | 0.0 | 2400 | 4.0 | 4.0 |
| 6(b)[2] | 25 | 0.0 | - | - | - |
| 7 | 25 | 1.0 | 2400 | 4.0 | 4.0 |
| 8 | 25 | 0.0 | - | - | - |
| 9 | 25 | 3.0 | 2400 | 1.0 | 1.0 |
| Untreated Check | - | 4.0 | - | 4.0 | 4.0 |

1. Average of two to four replicates
2. 6(a)=5-nitro, 6(b)=3-nitro

CLAIMS:

1. A nematicidal composition characterized by a nematicidal amount of a compound of the formula

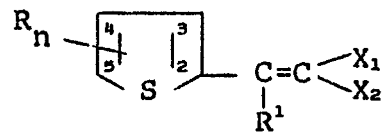

where each X is a halogen atom, each R is hydrogen, halogen, lower alkyl or nitro; n is 0, 1 or 2; and $R^1$ is hydrogen, lower alkyl, or di(lower)alkyl phosphate in admixture with an agriculturally acceptable carrier, diluent or extender.

2. The composition of claim 1 characterized in that at least one R is at the 3 or 5 position and is other than hydrogen.

3. The composition of claim 2 characterized in that R is a lower alkyl or nitro group at the 5 position.

4. The composition of claim 3 characterized in that the compound is 2-(2,2-dichloroethenyl)-5-methylthiophene.

5. The composition of claim 3 characterized in that the compound is 2-(2,2-dichloroethenyl)-5-nitrothiophene.

6. The composition of claim 1 characterized in that the compound is 2-(2,2-dichloroethenyl)-thiophene or 2-(2,2-dichloro-1-methylethenyl)thiophene or 2,2-dichloro-1-(2-thienyl)ethenyl diethylphosphate.

7. The composition of claim 1 characterized in that when R is hydrogen at least one R group on the thiophene ring is a nitro group.

8. A compound of the formula

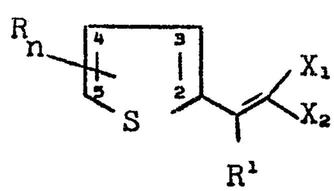

wherein each X is a halogen atom; each R is hydrogen, halogen, lower alkyl or nitro; n is 0, 1 or 2; $R^1$ is hydrogen, lower alkyl, or di(lower)alkyl phosphate, with the proviso that when $R^1$ is hydrogen at least one R group on the thiophene ring is nitro.

9. The compound of claim 8 characterized in that $R^1$ is hydrogen and at least one R group is nitro at the 3 or 5 position of the ring.

10. The compound of claim 9, 2-(2,2-dichloroethenyl-3-nitrothiophene.

11. The compound of claim 9, 2-(2,2-dichloroethenyl)-5-nitrothiophene.

12. The compound of claim 8 characterized in that $R^1$ is other than hydrogen.

13. The compound of claim 12, 2-(2,2-dichloro-1-methyl ethenyl)thiophene.

14. The compound of claim 12, 2,2-dichloro-1-(2-thienyl)ethenyl diethylphosphate.

15. A method for control of nematodes in agricultural crops characterized by applying to the locus of infestation a nematicidal amount of any of the nematicidal compositions defined in any of claims 1 to 7 or a compound defined in any of claims 8 to 14.